# EUROPÄISCHE PATENTSCHRIFT

(11) **EP 2 252 883 B1**
(45) Veröffentlichungstag und Bekanntmachung des Hinweises auf die Patenterteilung: **02.04.2014**
(21) Anmeldenummer: 08715559.4
(22) Anmeldetag: 10.03.2008
(51) Int. Cl.: G01N 27/64, G01N 33/00

(54) **VERFAHREN ZUR DETEKTION UND IDENTIFIZIERUNG VON GASEN IN FLUGZEUGINNENRÄUMEN**
METHOD FOR DETECTING AND IDENTIFYING GASES IN AIRPLANE INTERIOR SPACES
PROCÉDÉ DE DÉTECTION ET D'IDENTIFICATION DE GAZ DANS LES ESPACES INTÉRIEURS D'AÉRONEF

(43) Veröffentlichungstag der Anmeldung: 24.11.2010
(73) Patentinhaber: AIRSENSE Analytics GmbH, 19061 Schwerin (DE)
(72) Erfinder: WALTE, Andreas, 19059 Schwerin (DE); MÜNCHMEYER, Wolf, 38468 Ehra-Lessien (DE); SCHMIDT, Mario, 19053 Schwerin (DE)
(74) Vertreter: Gulde Hengelhaupt Ziebig & Schneider
(86) Internationale Anmeldenummer: PCT/DE2008/000422
(87) Internationale Veröffentlichungsnummer: WO 2009/112001

(56) Entgegenhaltungen:
- DE-A1- 10 247 272
- N. L. NAGDA, H. E. RECTOR: "A critical review of reported air concentrations of organic compounds in aircraft cabins" INDOOR AIR, Bd. 13, 2003, Seiten 292-301, XP002504842
- H. MUIR: "Cabin Air Sampling Study Functionality Test" CRANFIELD UNIVERSITY, [Online] November 2007 (2007-11), Seiten 1-5, XP002504843 Gefunden im Internet: URL:http://www.dft.gov.uk/pgr/aviation/hci /cabinairsamplingstudy.pdf> [gefunden am 2008-11-18]
- PALMER P T ET AL: "Mass spectrometry in the U.S. space program: past, present, and future" JOURNAL OF THE AMERICAN SOCIETY FOR MASS SPECTROMETRY, ELSEVIER SCIENCE INC, US, Bd. 12, Nr. 6, 1. Juni 2001 (2001-06-01), Seiten 656-675, XP004243274 ISSN: 1044-0305
- P ..V JOHNSON, L. W. BEEGLE, H. I. KIM, G. A. EICEMAN, I. KANIK: "Ion mobility spectrometry in space exploration" INTERNATIONAL JOURNAL OF MASS SPECTROMETRY, Bd. 262, 18. Dezember 2006 (2006-12-18), Seiten 1-15, XP002504844
- RICARDO GUTIERREZ-OSUNA: "Pattern Analysis for Machine Olfaction: A Review", IEEE SENSORS JOURNAL, IEEE SERVICE CENTER, NEW YORK, NY, US, vol. 2, no. 3, 1 June 2002 (2002-06-01), XP011065654, ISSN: 1530-437X
- AMPUERO S ET AL: "The electronic nose applied to dairy products: a review", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 94, no. 1, 15 August 2003 (2003-08-15), pages 1-12, XP004438553, ISSN: 0925-4005, DOI: 10.1016/S0925-4005(03)00321-6
- Stanislaw Osowski: "Hybrid Neural Network for Gas Analysis Measuring System", , 1 January 1999 (1999-01-01), XP055048075, Retrieved from the Internet: URL:http://ieeexplore.ieee.org/ielx5/6300/ 16856/00776791.pdf?tp=&arnumber=776791&isn umber=16856 [retrieved on 2012-12-17]
- KOLEHMAINEN M ET AL: "MONITORING ODOROUS SULFUR EMISSIONS USING SELF-ORGANIZING MAPS FOR HANDLING ION MOBILITY SPECTROMETRY DATA", JOURNAL OF THE AIR AND WASTE MANAGEMENT ASSOCIATION, AIR AND WASTE MANAGEMENT ASSOCIATION, PITTSBURGH, US, vol. 51, no. 7, 1 January 2001 (2001-01-01), pages 966-971, XP008028407, ISSN: 1047-3289
- Zvi Boger ET AL: "Use of Neural Networks for Quantitative Measurements in Ion Mobility Spectrometry (IMS)", J. Chem. Inf. Comput. Sci., vol. 34, 1 May 1994 (1994-05-01), pages 576-580, XP055061454,
- DAQI ET AL: "Simultaneous estimation of odor classes and concentrations using an electronic nose with function approximation model ensembles", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 120, no. 2, 22 December 2006 (2006-12-22), pages 584-594, XP005812369, ISSN: 0925-4005, DOI: 10.1016/J.SNB.2006.03.017
- Timothy R. Mcjunkin ET AL: "TOWARD AN INTELLIGENT ION MOBILITY SPECTROMETER (IMS)", International Journal for Ion Mobility Spectrometry, vol. 6, no. 2, 1 January 2003 (2003-01-01) , pages 31-39, XP055061471,

## Beschreibung

Die Erfindung bezieht sich auf ein Verfahren zur Detektion und Identifizierung von Gasen in Flugzeuginnenräumen nach dem Oberbegriff des Anspruchs 1.

Derartige Verfahren zur Detektion und Identifizierung von Gasen in Flugzeuginnenräumen werden zur Erkennung und zum Nachweis von Gasen, insbesondere von Gerüchen und explosiven und/oder gesundheitsschädigenden Gasen, eingesetzt.

Gerüche sind riechbare Gase. Da der Geruchseindruck subjektiv und von Mensch zu Mensch unterschiedlich ist, wird ein Geruchsschwellwert eingeführt, bei dem mehr als 50 % der Probanden den Geruchsstoff wahrnehmen. Die Geruchsstoffkonzentrationen am Geruchschwellwert sind für eine Reihe von Geruchsstoffen ermittelt und tabelliert wurden (z.B. in: Mills, B.: Review of Methods of Odour Control, Filtration & Separation 2 (1995), S. 147-152).

Die Detektion und Identifizierung von Gerüchen und explosiven und/oder gesundheitsschädigenden Gasen erfordert Messverfahren mit Nachweisgrenzen im ppt-ppb-Bereich. Zur Erkennung und zum Nachweis dieser Gase werden deshalb häufig Spektrometer eingesetzt. Dabei ist die Verwendung von Ionenmobilitätsspektrometern, die auch als Plasmä-Chromatographen bezeichnet werden, zu bevorzugen, weil sie im Gegensatz zu anderen Spektrometern, wie z.B. einem Massenspektrometer, keine Vakuumpumpe zur Erzeugung eines Vakuums für die Detektion der Gase benötigen. Deshalb sind Ionenmobilitätsspektrometer gegenüber anderen Spektrometern in ihrer Bauausführung klein und kostengünstig.
Der Anwendungsbereich der Ionenmobilitätsspektrometer ist sehr groß. Er reicht vom medizinischen Bereich, z.B. bei der Untersuchung der Ausatemluft von Patienten, über den Einsatz zur Produktionsüberwachung, z.B. bei der Qualitätskontrolle in einer Kaffeerösterei, bis zum militärischen Bereich, z.B. beim Nachweis von Kampfstoffen. Eine allgemeine Übersicht über Ionenmobilitätsspektrometer und deren Anwendungen finden sich beispielsweise in: G.A. Eiceman und Z. Karpas "Ion Mobility Spectrometry" (2nd. Edition, CRC, Boca Raton, 2005).

Auch können Gerüche und explosive und/oder gesundheitsschädigende Gase teilweise auch mit Messsystemen, welche hauptsächlich aus einzelnen Gasdetektoren oder Kombinationen aus verschiedenen Gasdetektoren bestehen, erfasst werden. Die Messsignale der einzelnen Gasdetektoren können dann mit vorher gemessenen bzw. auch gespeicherten Signalen verglichen und der gemessene Zustand beschrieben werden. Als Detektoren sind z. B. Photoionisierungsdetektoren, elektrochemische Zellen und Metalloxidsensoren einsetzbar. Die Verwendung von Messgeräten, die zweidimensionale Informationen, also Spektren, liefern, ist auch denkbar. Beispiele hierfür sind Massenspektrometer, FourierTransform-Infrarot-Spektrometer oder Ionen-Mobilitäts-Spektrometer.
Einfache Detektoren, wie z. B. Photoionisierungsdetektor, elektrochemische Zelle und Metalloxidsensor, sind mit ihren Nachweisgrenzen im oberen ppb- bzw. unteren ppm-Bereich zum Nachweis von einigen Gasen zu unempfindlich. Außerdem reicht ihre Selektivität häufig nicht aus, um die Schadstoffe mit der notwendigen Sicherheit nachzuweisen.

Aus der US 2959677 sind die wesentlichen Funktionsmerkmale eines Photoionisationsdetektors bekannt. Mittels einer UV-Lampe wird das nachzuweisende Gas ionisiert und anschließend elektrisch nachgewiesen. Ausschlaggebend ist in erster Linie das Ionisierungspotential der nachzuweisenden Verbindung. Falls die Energie der UV-Strahlung höher als die Ionisierungsenergie der Verbindung ist, lassen sich diese detektieren. Nachteilig ist, dass viele Schadstoffe nicht erfasst werden können. Es wird keine spektrale Information geliefert. Weiterhin ist nachteilig, dass Lampen des Photoionisationsdetektors kontaminieren können, was zu schlechteren Signalausbeuten führt.
Aus der US3631436 sind die wesentlichen Funktionsmerkmale der Metalloxidsensoren bekannt. Diese Sensoren reagieren mit reduzierenden und oxidierenden Gasen. Sie haben eine relativ starke Querempfindlichkeit und können nicht zur Einzelstofferkennung oder als Warnmelder wegen der großen Fehlalarmrate eingesetzt werden. Metalloxidsensoren zeichnen sich durch sehr schnelle Ansprechzeiten nach einer Gasexposition aus, haben aber den Nachteil, dass die Abfallzeiten wesentlich länger ausfallen.
Elektrochemische Zellen sind selektiver als Metalloxidsensoren. Eine Bestimmung von Einzelstoffen ist mit den Detektoren trotzdem nicht möglich, da auch hier Querempfindlichkeiten auftreten bzw. keine elektrochemischen Zellen für alle Substanzen zur Verfügung stehen. Aus der US3925183 sind die wesentlichen Funktionsmerkmale der elektrochemischen Zellen bekannt.
Das Ionenmobilitätsspektrometer oder der Plasma-Chromatograph ist seit längerem bekannt. Im Gegensatz zu anderen Spektrometern werden keine beweglichen oder aufwendigen Einzelteile im Ionenmobilitätsspektrometer benötigt, so dass diese Systeme klein und kostengünstig aufzubauen sind. Eine Beschreibung der einzelnen Komponenten in einem Ionenmobilitätsspektrometer findet sich z.B. in der US 3621240. Die unterschiedliche Beweglichkeit von Ionen wird beim Ionenmobilitätsspektrometer ausgenutzt. Diese Geräte bestehen aus einem Einlass-System, einer Ionenquelle, wobei typischerweise radioaktive Ni63-Folien eingesetzt werden, einer elektrischen Driftröhre, wo die Ionen nach einem definierten Start mittels eines elektrisch geschalteten Gitters nach ihrer Beweglichkeit bei Umgebungsdruck getrennt werden und einem Messfühler zum Nachweis der geringen elektrischen Ströme, die durch die auftreffenden Ionen erzeugt werden. In der Ionenquelle werden bei Atmosphärendruck hauptsächlich Luftmoleküle ionisiert, die anschließend Wassercluster ionisieren, welche auch Reaktant-Ionen genannt werden. Über Protonentransfer-, Elektronentransfer- oder Protonenabstraktions-Reaktionen werden anschließend die Schadstoffe ionisiert. Durch Änderung der Polarität der Driftstrecke können dann im positiven Betriebsmodus positive bzw. im negativen Betriebsmodus negative Ionen nachgewiesen werden.
Bei mobilen Systemen besteht der Einlass in der Regel aus einer Membran. In der US 4311669 wird ein Membraneinlasssystem für Ionenmobilitätsspektrometer beschrieben. Vorteilhaft ist, dass durch die Membran die Einflüsse des Messsignals durch Störgrößen wie z.B. Feuchte, Druck und Temperatur reduziert werden und dass dadurch IMS-Systeme klein und tragbar herzustellen sind. Nachteilig ist, dass die Membran die Messsysteme in ihrer Antwortzeit etwas träger reagieren lässt.

Aus der DE 102 35 612 A1 ist ein Verfahren und eine dazugehörige Vorrichtung zur Überwachung der Qualität von Schmierölen bekannt, bei dem aus einem Getriebe eine Probe des Schmieröls oder des aus dem Schmieröl austretenden Dampfes entnommen wird, diese Probe anschließend einem Ionenmobilitätsspektrometer zugeführt und damit die Probe auf in der Dampfphase über dem Schmieröl vorhandene Stoffe analysiert wird. Dabei sollen die Veränderungen des Gehaltes und der Art der Stoffe in der Probe analysiert und gegenüber vorbestimmten Stoffen in der Dampfphase des ungebrauchten Schmieröls als Ist-Zustand für die Alterung des Schmieröles durch Vergleich ausgewertet werden. Dazu ist vorrichtungsseitig das Ionenmobilitätsspektrometer einerseits über eine Probeentnahmeleitung mit dem Getriebe und andererseits mit einer Auswerteeinheit verbunden.
Von Nachteil an dieser Lösung ist aber, dass das Verfahren und die dazugehörige Vorrichtung ausschließlich zur Überwachung der Qualität von Schmierölen in Getrieben anwendbar sind, weil diese Vorrichtung nur auf Schmieröle von Getrieben kalibriert ist. Eine Detektion, also Erkennung des untersuchten Stoffes, wenn dieser kein Schmieröl von Getrieben ist, ist damit nicht möglich. Damit ist auch ein Einsatz dieser Lösung in einem Flugzeuginnenraum und/oder zur Untersuchung von Gasen eines Flugzeuges, insbesondere zur Untersuchung des Wartungszustandes von Flugzeugtriebwerken, ungeeignet.
Besonders nachteilig bei den Ionenmobilitätsspektrometern ist außerdem die lange Zeit, die abzuwarten ist, bis nach dem Einschalten des Gerätes das System wieder messbereit ist. Der Detektor des Ionenmobilitätsspektrometers benötigt diese Zeit, da er Störsubstanzen, die sich während des ausgeschalteten Zustandes angereichert haben, aus dem System spülen muss. Nachteilig ist weiterhin, dass bei kurzfristiger Überdosierung die Systeme nicht mehr messbereit sind und mehrere Minuten, bis hin zu Stunden, gespült werden müssen. Störend ist weiterhin, dass die Spektren konzentrationsabhängig sind.
Ein weiteres Problem ist die teilweise geringe Selektivität des Ionenmobilitätsspektrometers. Ein Grund dafür ist, dass durch konkurrierende Reaktionen im Ionisierungsraum häufig nicht die interessierenden Schadstoffe ionisiert werden und somit nicht nachzuweisen sind. Diese konkurrierenden Reaktionen können dazu führen, dass z.B. bei Anwesenheit von Gasen wie z.B. Ammoniak viele Schadstoffe mit geringerer Protonenaffinität, wie z.B. viele Lösungsmittel, gar nicht mehr im Spektrum erscheinen.
Im Gegenzug kann dann aber durch Anwesenheit von Lösungsmitteln in höheren Konzentrationen (ppm) der Nachweis von den interessierenden Verbindungen erschwert, wenn nicht sogar unmöglich werden. Durch die überlagerten Spektren in einem Gemisch aus Gasen wird dann die Fehlalarmrate gesteigert.
Weiterhin werden Schadstoffe mit geringer Protonenaffinität oder Elektronenaffinität nicht mit den geforderten Nachweisgrenzen ermittelt.
Ein weiterer Nachteil des Ionenmobilitätsspektrometers ist der begrenzte Messbereich, der z.B. bei einem Beta-Strahler als Ionisierurigsquelle typischerweise maximal zwei Größenordungen beträgt. Eine quantitative Aussage wird dadurch schwierig.

Aus DE 102 47 272 A1 ist ein Verfahren und eine Vorrichtung zur Detektion von Gasen in Flugzeuginnenräumen bekannt, bei der die Zuluft des Flugzeuginnenraumes einer ein Ionenmobilitätsspektrometer umfassenden Messvorrichtung zugeführt wird.

Der Erfindung liegt daher die Aufgabe zu Grunde, ein gattungsgemäßes Verfahren zur Detektion und Identifizierung von Gasen in Flugzeuginnenräumen zu entwickeln, das eine sofortige und zeitgleiche Detektion und Identifizierung der zu untersuchenden Gase zulässt.

Diese Aufgabe wird durch ein Verfahren mit den in Anspruch 1 genannten Merkmalen gelöst.

Das erfindungsgemäße Verfahren zur Detektion und Identifizierung von Gasen in Flugzeuginnenräumen beseitigt die genannten Nachteile des Standes der Technik.
Vorteilhaft bei der Anwendung des neuen Verfahrens zur Detektion und Identifizierung von Gasen in Flugzeuginnenräumen ist es, dass die Zuluft des Flugzeuginnenraumes einer Messvorrichtung zugeführt wird und die Messergebnisse der Messvorrichtung mittels mathematischer Methoden analysiert werden. Dadurch wird eine sofortige und zeitgleiche Detektion und Identifizierung der zu untersuchenden Gase ermöglicht.
Von Vorteil ist dann, wenn die mathematischen Methoden sich in eine Kalibrierung und eine Gasvermessung unterteilen, wobei die Kalibrierung eine Aufteilung des Messbereiches der Messvorrichtung in Gasklassen und eine Zuordnung von Grenzwerten für jede Gasklasse sowie die Gasvermessung eine Erkennung der Gasklassen und eine Ermittlung der Gasintensität umfasst. Dabei wird die Kalibrierung bei für die Messvorrichtung unbekannten Gase verwendet, wobei die Messvorrichtung auf die unbekannten Gase trainiert wird und die Zuordnung von Grenzwert für jede Gasklasse eine Datenbank mit einer paarweisen Anordnung von verschiedenen Messsignalen der Messvorrichtung und Grenzwerten erstellt wird. Dabei ist dieses Verfahren auch auf unbekannte Gase anwendbar, weil die unbekannten Gase trainierbar werden können. Dadurch ist der Einsatzbereich leicht erweiterbar.
Von besonderem Vorteil ist dann, wenn bei riechbaren Gasen die Kalibrierung der Messvorrichtung für unbekannte Gerüche erfolgt, wobei die Zuordnung von Grenzwert für jede Gasklasse eine Datenbank mit einer paarweisen Anordnung von verschiedenen Messsignalen der Messvorrichtung und Geruchsschwellwerten erstellt wird. Dabei wird durch die Verwendung von Geruchsschwellwerten sichergestellt, dass dieses Verfahren bei der Untersuchung von Geruchbelästigungen einsetzbar ist.
Vorteilhaft ist auch, wenn bei der Gasvermessung mit der Erkennung der Gasklassen eine Gasklasse ausgewählt wird, für die anschließend die Ermittlung der Gasintensität durchgeführt wird, wobei bei der Gasvermessung die ermittelten Messergebnisse der Messvorrichtung mit den vorher aufgenommenen Messergebnissen verglichen und aus dem Vergleich Rückschlüsse auf das vermessene Gas gezogen werden.
Von besonderem Vorteil ist es, wenn eine Änderung des Ansaugkanals der Zuluft des Flugzeuginnenraumes, oder durch eine Änderung der Betriebsparameter vor dem Ansaugkanal, Rückschlüsse auf Gasquellen des vermessenen Gases im Ansaugbereich des Ansaugkanals geschlossen wird, wobei die Rückschlüsse insbesondere die Art der Gasquelle und der Ort der Gasquelle sind. Durch die Änderung des Ansaugkanals der Frischluft des Flugzeuginnenraumes, oder durch eine Änderung der Betriebsparameter eines Flugzeugtriebwerkes, können beispielsweise Rückschlüsse auf den Wartungszustand eines im Ansaugbereich des Ansaugkanals befindlichen Flugzeugtriebwerkes geschlossen werden. Dadurch ist eine einfache und sicherere Art der Lokalisierung der Gasquelle gegeben. Da üblicherweise der größte Teil der Luftmenge der Lüftung des Flugzeuginnenraumes im Kreislauf gefahren wird, kann durch gezielte Absperrung der Abluftkanäle des Flugzeuginnenraumes, bei gleichzeitiger Absperrung der Frischluftzufuhr und damit im Umluftbetrieb der Lüftung, eine Gas- oder Geruchsquelle innerhalb des Flugzeuginnenraumes lokalisiert oder auch ausgeschlossen werden. So lassen sich neben Geruchsquellen auch Brände im Flugzeuginnenraum, insbesondere Kabelbrände unter der Verkleidung des Flugzeuginnenraumes, lokalisieren. Der restliche Teil der Luftmenge der Lüftung des Flugzeuginnenraumes, welche nicht im Kreislauf gefahren wird, ist Frischluft, die über die Flugzeugtriebwerke in den Flugzeuginnenraum gelangt und woraus durch eine Änderung des Ansaugkanals der Frischluft des Flugzeuginnenraumes Rückschlüsse auf den Wartungszustand eines im Ansaugbereich des Ansaugkanals befindlichen Flugzeugtriebwerkes geschlossen werden können. Wenn dabei die Gas- oder Geruchsintensität des vermessenen Gases beispielsweise den Geruchsschwellwert oder einen Grenzwert unterhalb oder oberhalb des Geruchsschwellwertes überschritten hat und dadurch eine erhebliche Geruchsbelästigung der im Flugzeuginnenraum befindlichen Passagiere zu erwarten ist, wird der Ansaugkanal dieses Flugzeugtriebwerkes und das lokalisierte Flugzeugtriebwerk beim nächsten Stillstand des Flugzeuges einer Wartung unterzogen und dabei die entsprechende Leckage abgedichtet.
Von Vorteil ist weiter, wenn die Aufteilung des Messbereiches der Messvorrichtung in Gasklassen durch ein künstlich-neuronales Netz, insbesondere durch ein Kohonen-Netz, und die Zuordnung von Grenzwerten für jede Gasklasse durch ein künstlich-neuronales Netz, insbesondere durch ein Multilayer-Perceptron-Netz, erfolgen. Dabei sind diese künstlich-neuronalen Netze besonders gut zur Mustererkennung geeignet und gewährleisten deshalb nur eine geringe Messunsicherheit.
Auch ist von Vorteil, wenn das zu messende Gas durch eine Förderpumpe angesaugt und durch Verteiler aufgeteilt wird, dann parallel einem Ionenmobilitätsspektrometer, einer elektrochemischen Zelle, einem Photoionisationsdetektor und/oder zwei Metalloxidsensoren zugeführt wird. Dadurch werden die Bandbreite der erkennbaren Gase und damit die Einsatzbreite weiter erweitert, so dass diese Lösung auch auf die verschiedensten Gasgemische anwendbar ist. Dabei können gleichzeitig verschiedene, in dem untersuchten Gasgemisch vorhandene Gase detektiert und identifiziert werden.
Von besonderem Vorteil ist es dann, wenn das zu messende Gas vor dem Erreichen des Ionenmobilitätsspektrometers, der elektrochemischen Zelle, des Photoionisationsdetektors und der Metalloxidsensoren, alternativ auch nur vor dem Erreichen des Ionenmobilitätsspektrometers, mit einem Referenzgas vermischt wird. Dazu wird das Referenzgas in definierten Mengen dem zu messenden Gas zugeführt, wobei beispielsweise mit der höchstmöglichen Flussmenge des Referenzgases angefangen wird und diese später reduziert wird. Dabei kann das Messsignal des Ionenmobilitätsspektrometers, der elektrochemischen Zelle, des Photoionisationsdetektors und/oder der Metalloxidsensoren zur Regelung der Dosierung des Referenzgases verwendet werden, indem die relative Signalhöhe der Metalloxidsensoren direkt genutzt wird, um den Gasfluss der Förderpumpe kurzfristig zu erhöhen, falls die Messsignale der Metalloxidsensoren zunehmen sollten, oder um den Gasfluss der Förderpumpe kurzfristig zu verringern, falls das Messsignal des Detektors abnehmen sollte, und/oder aus dem Messsignal des Ionenmobilitätsspektrometers die absoluten Bereiche der Gasflussmenge des Referenzgases einzustellen, indem die absoluten Signalhöhen während eines längeren Zeitraumes verwendet werden, um die grobe Einstellung insbesondere eine Einstellung des maximalen und minimalen Bereichs der Fördermenge des Referenzgases des Gasflusses zu ermöglichen. Dadurch werden die Detektoren einerseits geschont, weil eine Vergiftung der Detektoren vermieden wird. Andererseits lässt sich durch diese Lösung der Messbereich erweitern, so dass die Einsatzbreite zusätzlich erweitert wird. Das Referenzgas kann zum Beispiel aus Aktivkohle-gefilterter Luft bestehen, kann aber auch aus gereinigter Luft mit speziellen Substanzen, so genannten Dotiergasen, bestehen.
Von Vorteil ist auch, wenn das zu messende Gas über eine Kombination aus dem Ionenmobilitätsspektrometer und dem Photoionisationsdetektor zum Nachweis von Aromaten, und zusätzlich mit der elektrochemischen Zelle zum Nachweis einzelner Substanzen, wie z.B. Phosgen, und zusätzlich zu den Metalloxidsensoren zum Nachweis von z.B. Kohlenwasserstoffen oder Kohlenmonoxid geleitet werden.

Die Erfindung soll nun an drei Ausführungsbeispielen erläutert werden.

Dazu zeigen:
- Fig. 1:: eine schematische Darstellung einer Vorrichtung in einem ersten Ausführungsbeispiel,
- Fig.. 2:: eine schematische Darstellung einer Vorrichtung in einem zweiten Ausführungsbeispiel,
- Fig. 3:: eine schematische Darstellung einer Vorrichtung in einem dritten Ausführungsbeispiel und
- Fig. 4:: eine vereinfachte Darstellung der Messanordnung und ein Blockschaltbild der Messung eines Gases in einem Flugzeuginnenraum.

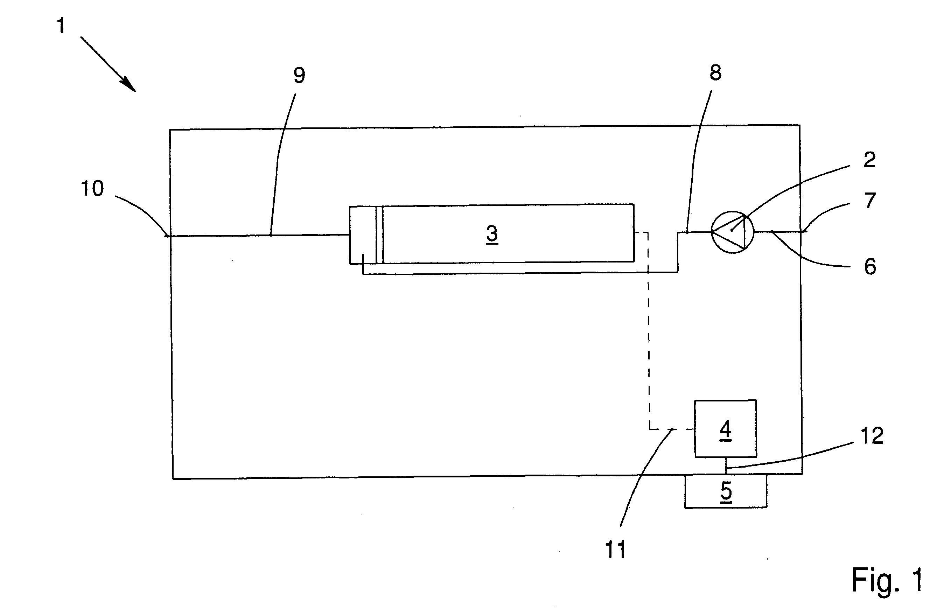

Die Vorrichtung zur Identifizierung von Gasen besteht in einem ersten Ausführungsbeispiel gemäß der **Fig. 1** im Wesentlichen aus einer Messvorrichtung 1 mit einer Förderpumpe 2, einem Ionenmobilitätsspektrometer 3 und einem elektronischen Rechner 4 mit einer Bedien- und Anzeigeeinheit 5.
Dabei ist die Förderpumpe 2 druckseitig über einen Gasleitung 6 mit einem Abluftstutzen 7 gegenüber der Atmosphäre offen. Saugseitig ist die Förderpumpe 2 über eine Gasleitung 8 ausgangsseitig mit dem Ionenmobilitätsspektrometer 3 verbunden, wobei das Ionenmobilitätsspektrometer 3 eingangsseitig wiederum über eine Gasleitung 9 mit einem Ansaugstutzen 10 verbunden ist.

Weiterhin ist der Rechner 4 über eine Leitung 11 elektrisch mit dem Ionenmobilitätsspektrometer 3 und über eine Leitung 12 elektrisch mit der Bedien- und Anzeigeeinheit 5 verbunden.

Die Bedien- und Anzeigeeinheit 5 bietet eine graphisch und akustisch Darstellung der Messergebnisse. Daneben besitzt die Bedien- und Anzeigeeinheit 5 typische Bedienelemente wie beispielsweise einen Ein-/Ausschalter oder eine Start-/Stopp-Taste zur manuellen Steuerung eines Messzyklus.

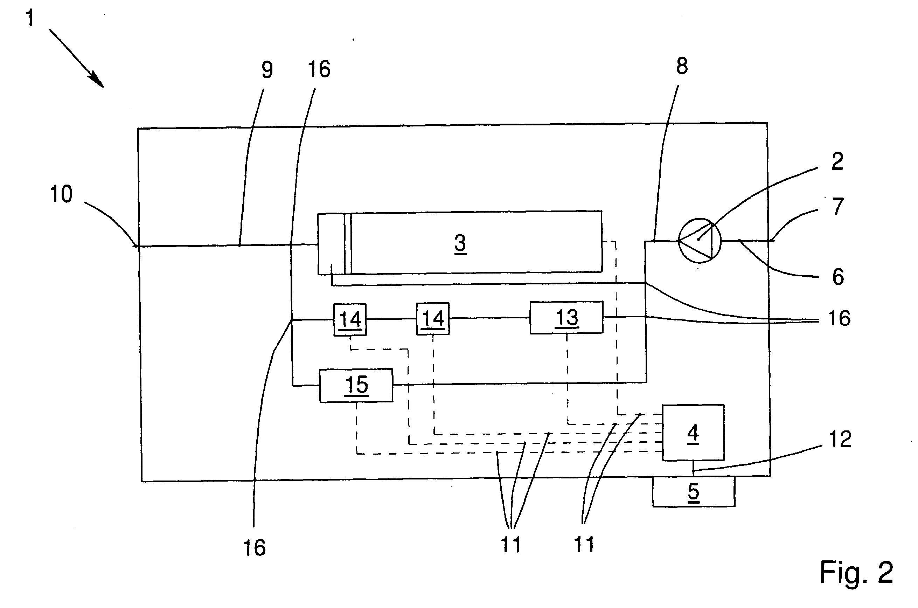

Die Vorrichtung zur Identifizierung von Gasen weist in einem zweiten Ausführungsbeispiel gemäß der **Fig. 2** zusätzlich ein Photoionisationsdetektor 13, zwei Metalloxidsensor 14 und eine elektrochemische Zelle 15 auf.
Dabei sind der Photoionisationsdetektor 13 zusammen mit dem Metalloxidsensor 14 und die elektrochemische Zelle 15 strömungstechnisch jeweils parallel zu dem Ionenmobilitätsspektrometer 3 angeordnet. Dazu weisen die Strömungskanäle 8 und 9 jeweils zwei Verteiler 16 auf. Weiterhin sind der Photoionisationsdetektor 13, die Metalloxidsensoren 14 und die elektrochemische Zelle 15 elektrisch über eine weitere Leitung 11 mit dem Rechner 4 verbunden.
Denkbar ist es auch, anstelle des Metalloxidsensors 14 ein Sensorarray mit gleichen und/oder verschiedenen Metalloxidsensoren 14 zu verwenden.

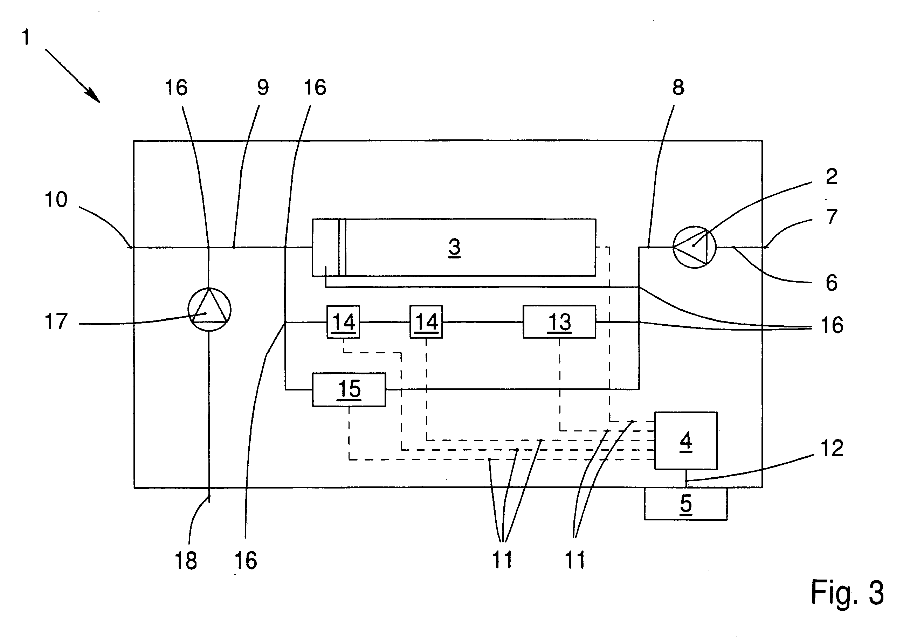

Die Vorrichtung zur Identifizierung von Gasen weist in einem dritten Ausführungsbeispiel gemäß der **Fig. 3** zusätzlich eine Förderpumpe 17 zur kontrollierten Dosierung eines Referenzgases auf, die über einen weiteren Verteiler 16, direkt hinter den Ansaugstutzen 10 in die Gasleitung 9 mündet und mit der über den Ansaugstutzen 18 ein Referenzgas dem Ionenmobilitätsspektrometer 3, Photoionisationsdetektor 13, Metalloxidsensor 14 und der elektrochemische Zelle 15 zugeführt werden kann.
Denkbar ist auch, die in Fig.3 gezeigt Förderpumpe 17 zur kontrollierten Dosierung eines Referenzgases nur in Kombination mit dem Ionenmobilitätsspektrometer, ohne zusätzliche Sensoren, zu verwenden.

Auf dem Rechner 4 sind bei allen zuvor genannten Ausführungsbeispielen Computerprogramme mit verschiedenen mathematischen Methoden installiert. Diese mathematischen Methoden umfassen eine Kalibrierung und eine Gasvermessung, wobei die Kalibrierung eine Aufteilung des Messbereiches der Messvorrichtung 1 in Gasklassen und eine Zuordnung von Grenzwerten für jede Gasklasse sowie die Gasvermessung eine Erkennung der Gasklassen und eine Ermittlung der Gasintensität umfassen. Dabei erfolgt die Aufteilung des Messbereiches der Messvorrichtung 1 in Gasklassen sowie Zuordnung von Grenzwerten für jede Gasklasse durch jeweils ein Künstlich-neuronales Netz, wobei für die Aufteilung des Messbereiches der Messvorrichtung 1 in Gasklassen insbesondere ein Kohonen-Netz und für die Zuordnung von Grenzwerten für jede Gasklasse insbesondere ein Multilayer-Perceptron-Netz einsetzbar ist. Prinzipiell ist aber auch jedes andere geeignete Künstlich-neuronale Netz oder jede andere mathematische Methode zur Mustererkennung und zur Quantifizierung einsetzbar.
Bei der Aufteilung des Messbereiches wird das gesamte Spektrum der mit der Messvorrichtung 1 zumessenden Gase in Gasklassen eingeteilt. Dazu wird das Kohonen-Netz eingesetzt. Diese Einteilung erfolgt in einer Trainings- oder Lemphase. In der Trainings- oder Lemphase werden die Gewichtsfunktion der einzelnen Neuronen des Kohonen-Netzes an einen Trainings- oder Lerndatensatz angepasst. Dazu werden mit der Messvorrichtung 1 bekannte zumessende Gase vermessen und die gewonnenen Messsignale, für jedes Gas zu Datensätzen zusammengefasst, abgespeichert. Aus den Datensätzen werden für das Kohonen-Netz durch einen Lernalgorithmus die Gasklassen berechnet, wobei jede Gasklasse jeweils ein zumessendes Gas interpretiert.
Bei der Zuordnung von Grenzwerten für jede Gasklasse wird mindestens ein Grenzwert jeder Gasklasse zugeordnet, wobei dazu das Multilayer-Perceptron-Netz verwendet wird. Diese Grenzwerte sind beispielsweise geruchsbelästigende und/oder gesundheitsschädigende Gaskonzentrationen. Dabei ist es auch denkbar, dass der Grenzwert bei riechenden Gase bzw. Gerüchen der Geruchsschwellwert ist.
Die Zuordnung von Grenzwerten zu jeder Gasklasse erfolgt wiederum in einer Trainingsoder Lemphase des Multilayer-Perceptron-Netzes, wobei diese Zuordnung für jedes Gas durchgeführt wird. Dazu werden mit der Messvorrichtung 1 vordefinierte Gaskonzentrationen des bekannten zumessenden Gases vermessen. Die gewonnenen Messsignale werden für jede Gaskonzentration zu Datensätzen zusammengefasst abgespeichert. Aus den Datensätzen für jedes Gas werden durch einen Lernalgorithmus die Zuordnung von Grenzwerte berechnet.
Als Lernalgorithmus für die Aufteilung des Messbereiches der Messvorrichtung 1 in Gasklassen und die Zuordnung von Grenzwerten für jede Gasklasse dient jeweils das Gauß'sche Fehlerquadrat, wobei auch jede andere geeignete Methode der Fehlerminimierung einsetzbar ist.

Die Funktionsweise der Vorrichtung zur Identifizierung von Gasen in Flugzeuginnenräumen soll beispielhaft am zweiten Ausführungsbeispiel erläutert werden.
Die Messvorrichtung 1, welches entsprechend **Fig. 2** ein Ionenmobilitätsspektrometer 3, ein Photoionisationsdetektor 13, zwei Metalloxidsensoren 14 und eine elektrochemische Zelle 15 aufweist, wird derart mit seinem Ansaugstutzen 10 vor den Zuluftauslass 19 der Luftzufuhr in einen Flugzeuginnenraum 20 positioniert, dass ausschließlich die Luft bzw. das Gas aus dem Zuluftauslass 19 in den Ansaugstutzen 10 gelangt.
Während des Messvorganges wird das zu messende Gas über die Förderpumpe 2 angesaugt und gelangt dabei über den Ansaugstutzen 10, der Gasleitung 9 und die Verteiler 16 jeweils zum Ionenmobilitätsspektrometer 3, zur elektrochemischen Zelle 15 und nacheinander zum Photoionisationsdetektor 13 und den Metalloxidsensoren 14. Strömungstechnisch werden diese einzelnen Gasströme hinter dem Ionenmobilitätsspektrometer 3, der elektrochemischen Zelle 15 und dem Photoionisationsdetektor 13 über die Verteiler 16 wieder zusammengeführt und sind über den Gasleitung 9 mit der Förderpumpe 2 verbunden.

Der elektronische Rechner 4 bereitet die Messdaten des Ionenmobilitätsspektrometers 3, der elektrochemischen Zelle 15, des Photoionisationsdetektors 13 und der Metalloxidsensoren 14 einzeln auf und stellt diese über die Bedien- und Anzeigeeinheit 5 graphisch oder akustisch dar.
Beispielsweise können beim Ionenmobilitätsspektrometer 3 nur die integrierten Messsignale vor und nach dem Reaktant-Ionenpeak verwendet werden. Da das System sowohl im negativen wie auch im positiven Betriebsmodus arbeiten kann, ergeben sich somit vier Messkanäle. Zusammen mit den Signalen der elektrochemischen Zelle 15, des Photoionisationsdetektors 13 und der Metalloxidsensoren 14 stehen in der oben geschilderten Konfiguration acht Messkanäle zur Verfügung. Gegebenenfalls lassen sich die Spektren des Ionenmobilitätsspektrometers 3 auch feiner unterteilen, so dass wesentlich mehr als vier Kanäle zur Auswertung herangezogen werden können.
Die Kanäle können als Eingangssignale für eine folgende Mustererkennung verwendet werden. Denkbar ist auch die Auswertung der Ionenmobilitätsspektren derart, dass nur die auftretenden Peaks im Spektrum für eine folgende Auswertung herangezogen werden. Einfache Distanzklassifikatoren, wie z.B. der Abstandsklassifikator nach Euklid, bis hin zu Diskriminanzklassifikatoren oder auch neuronale Netze können hier eingesetzt werden. Eine Darstellung der Ionenmobilitätsspektren, bei der das Messsignal des Ionenmobilitätsspektrometers 3 als Funktion der Zeit dargestellt wird, ist auch möglich.

Gemäß dem dritten Ausführungsbeispiel gemäß **Fig. 3** kann die Messvorrichtung 1 über eine weitere Förderpumpe 17 zur Zumischung von einem Referenzgas in der Gasleitung 9 aufweisen, wobei das zugemischte Referenzgas ebenfalls von der oben aufgeführten Förderpumpe 2 angesaugt wird. Um das Ionenmobilitätsspektrometer 3, die elektrochemische Zelle 15, den Photoionisationsdetektor 13 und die Metalloxidsensoren 14 zu schonen, wird die Messvorrichtung 1 so betrieben, dass hauptsächlich das Referenzgas gemessen wird. Bei geringen Messsignalen wird die Fördermenge der Förderpumpe 17 reduziert, so dass der Anteil des zu messenden Gases zunimmt. Die Zudosierung des Referenzgases kann über definierte Stufen erfolgen oder aber auch durch das Messsignal eines Detektors geregelt werden.
Denkbar ist auch, dass das zumessende Gas alternativ nur vor dem Erreichen des Ionenmobilitätsspektrometers 3 mit einem Referenzgas vermischt wird und den anderen Detektoren, also der elektrochemischen Zelle 15, dem Photoionisationsdetektor 13 und den Metalloxidsensoren 14, das zumessende Gas in einem mit dem Referenzgas unverdünnten Zustand zuzuführen.

Es ist auch denkbar, die nicht im Einsatz befindliche Messvorrichtung 1 in einer speziellen Aufbereitungsstation zu lagern. Dort sorgt eine Förderpumpe in der Aufbereitungsstation dafür, dass die Messvorrichtung 1 ständig mit dem Referenzgas gespült wird. Dadurch wird verhindert, dass sich störende Komponenten in der Messvorrichtung 1 anlagern. Außerdem ist die Messvorrichtung 1 dann innerhalb von wenigen Minuten einsatzbereit. Die Aufbereitungsstation kann gleichzeitig auch zur Aufladung der Akkumulatoren und zum Auslesen der in der Messvorrichtung 1 gespeicherten Messdaten genutzt werden.

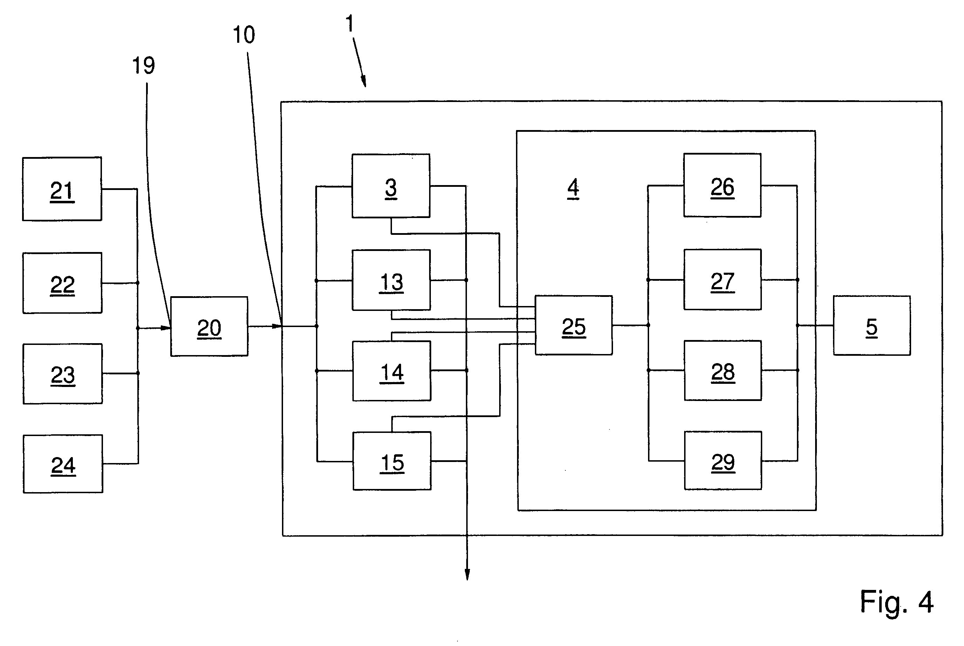

Bei der Anwendung der Vorrichtung zur Identifizierung von Gasen in Flugzeuginnenräumen lassen sich durch die Änderung der Zuluft des Flugzeuginnenraumes Rückschlüsse auf Gasquellen des vermessenen Gases im Ansaugbereich des Ansaugkanals ziehen. Dabei lassen sich insbesondere die Art der Gasquelle und der Ort der Gasquelle ermitteln.
Bei einer, gemäß der **Fig. 4****,** stationären Installation der Messvorrichtung 1 in den Zuluftauslass 19 der Lüftung des Flugzeuginnenraumes 20 läuft beim Auftreten einer Gasoder Geruchsbelästigung nachfolgender Prozess ab.
Die Zuluft in den Flugzeuginnenraum 20 wird mit den Ansaugstutzen 10 der Messvorrichtung 1 angesaugt, wobei die Zuluft eine Teil Frischluft enthalten soll, die über die Flugzeugtriebwerke 21, 22, 23, 24 zu gleichen Teilen in die Zuluft gelangt.
Beim Auftreten einer Gas- oder Geruchsbelästigung erfolgt mit der kalibrierten Messvorrichtung 1 bei der Gasvermessung im Rechner 4 zunächst einmal die Erkennung der Gasklasse. Damit erfolgt eine Einordnung des vermessenen Gases in eine Gasklasse. Beispielhaft können diese Gasklassen sein:
- Triebwerköle und -schmierstoffe (Gasklasse A),
- Kerosin (Gasklasse B),
- Abgase des Flugzeugtriebwerks (Gasklasse C),
- Brandgeruch, also hauptsächlich Kohlenmonoxid, Kohlendioxid und Verbindungen der unvollständigen Verbrennung (Gasklasse D).

Jeder dieser Gasklassen ist eine auf diese Gasklasse kalibrierte Ermittlung der Gas- oder Geruchsintensität hinterlegt. Dabei können natürlich beispielsweise die Triebwerköle und - schmierstoffe auch für sich in mehrere Gasklassen unterteilt werden. Für die zuvor beispielhaft genannten Gasklassen ergeben sich demnach vier verschiedene Auswertungen. Jede Gasklasse erfordert ihre Ermittlung der Gas- oder Geruchsintensität, also:
- Ermittlung der Gas- oder Geruchsintensität 26 für die Gasklasse A,
- Ermittlung der Gas- oder Geruchsintensität 27 für die Gasklasse B,
- Ermittlung der Gas- oder Geruchsintensität 28 für die Gasklasse C und
- Ermittlung der Gas- oder Geruchsintensität 29 für die Gasklasse D.
Wird das vermessene Gas beispielsweise in die Gasklasse A, als Triebwerköle und -schmierstoffe eingeordnet, kann zunächst einmal die Herkunft des vermessenen Gases mit den Flugzeugtriebwerken lokalisiert werden, so dass ein oder mehrere der Flugzeugtriebwerke 21, 22, 23, 24 in Betracht kommen. Bei Überschreiten des Geruchsschwellwert der Triebwerköle und -schmierstoffe entsteht unter Umständen eine erhebliche Geruchsbelästigung für die Passagiere.
Da die Kabine aufgrund der Größe nicht ausreichend gelüftet und von der Geruchsintensität befreit werden kann, lässt sich weder ein Triebwerk für die Geruchsbelästigung vermuten noch bestimmen. Die Ermittlung oder das Ausschlussverfahren der Flugzeugtriebwerke 21, 22, 23, 24 erfolgt anschließend, wobei die Ergebnisse für die Ermittlung der Gasklassen und Geruchsintensität an der Bedien- und Anzeigeeinheit 5 sofort angezeigt werden.
Dazu werden entweder die Flugzeugtriebwerke 21 und 24 oder die Flugzeugtriebwerk 22 und 23 auf gleiche Drehzahl gebracht. Wenn beispielsweise die Flugzeugtriebwerke 21 und 24 auf gleiche Drehzahl gebracht erfolgt nun die Zuschaltung der Zuluft für das Flugzeugtriebwerk 21 und die Abschaltung der Zuluft des Flugzeugtriebwerkes 24. Durch das Vermessen der Zuluft des Flugzeugtriebwerkes 21 durch die Messvorrichtung 1 kann geprüft werden, ob sich in der Zuluft Gasklassen und Geruchsintensitäten erkennen lassen. Dazu erfolgt zunächst einmal die Auswertung der Messsignale durch die Erkennung der Gasklassen 25. Wird beispielsweise Gasklassen A für "Triebwerköle und -schmierstoffe" erkannt, so erfolgt anschließend die Ermittlung der Gas- oder Geruchsintensität 26 für die Gasklasse A.
Anschließend erfolgen die Zuschaltung der Zuluft des Flugzeugtriebwerkes 24 und die Abschaltung der Zuluft des Flugzeugtriebwerkes 21. Zu diesem Zeitpunkt hätte unter Umständen die erste Wahrnehmung der Geruchsklasse durch das menschliche Organ stattfinden können, jedoch ist eine Zuordnung der Geruchsintensität unmöglich. Nach der ersten Wahrnehmung der Geruchsintensität durch die menschliche Nase bleibt dieser Geruchseindruck erhalten und weitere Wahrnehmungen der gleichen Geruchsklasse und Geruchsintensität sind unmöglich.
Nun erfolgt die Vermessung der Zuluft des Flugzeugtriebwerkes 24 durch die Messvorrichtung 1 mit dem Ziel, ebenfalls die Geruchsklasse und ggf. die Geruchsintensität zu bestimmen.
Anschließend wird mit den Flugzeugtriebwerken 22 und 23 gleichermaßen verfahren. Nach Abschluss dieser Methode kann durch die Auswertung der Ergebnisse der Messvorrichtung 1 eine Aussage darüber getroffen werden, in welcher Zuluft Gasklassen bestimmt werden konnten und mit welcher Geruchsintensität.
Denkbar ist auch, dass statt einer Zuschaltung, bzw. Abschaltung der Zuluft einzelner Triebwerke eine erhöhte Geruchsintensität dadurch erzielt wird, indem die einzelnen Triebwerke aus einem Leerlauf-Zustand auf höhere Drehzahlen gebracht werden, so dass dann die Konzentrationen der geruchstragenden Verbindungen erhöht werden. Defekte Triebwerke weisen dann wesentlich höhere Konzentrationen der Geruchsstoffe, bzw. Schmier- oder Hydrauliköle auf.
Die Geruchsintensitäten liegen oftmals unter der menschlich wahrnehmbaren Geruchsschwelle, so dass ein subjektiver Eindruck nicht zum gewünschten Erfolg führen würde. Durch das Zuführen der Zuluft über die Flugzeugtriebwerke 21, 22, 23, 24 und durch das kontinuierliche Vermessen der Zuluft durch die Messvorrichtung 1 kann die Zunahme oder Abnahme der Geruchsintensitäten erkannt und bestimmt werden.
Bei der Auswertung der Gasklassen und Geruchsintensitäten durch die Ergebnisse der Messvorrichtung 1 werden oft Geruchsintensitäten bestimmt, welche nicht zwangsläufig zu Fehlgerüchen im Flugzeuginnenraum 20 führen und/oder für die menschlichen Nase noch nicht wahrnehmbar sind. Diese Gas- oder Geruchsintensitäten liefern aber trotzdem Rückschlüsse auf den Triebwerkszustand, so dass sich bereits vor dem Entstehen von Geruchsintensitäten und der Wahrnehmung dieser durch die Passagiere feststellen lässt, ob in der Zukunft für bestimmte Flugzeugtriebwerke mit Geruchsklassen oder Geruchsintensitäten zu rechnen ist.

Bei einer stationären Installation der Messvorrichtung 1 in den Zuluftauslass 19 der Lüftung des Flugzeuginnenraumes 20 ist es auch denkbar, den Rechner 4 und/oder die Bedien- und Anzeigeeinheit 5 mit einer externen Bedien- und Anzeigeeinheit, die beispielsweise im Cockpit des Flugzeuges installiert ist, elektrisch zu verbinden.

Bei einer mobilen Messvorrichtung 1 kann durch Veränderung des Messortes zusätzlich der Ort der Gas- und Geruchsquelle eingeschränkt werden.
So lassen beispielsweise durch Veränderung des Messortes über die Verkleidung des Flugzeuginnenraumes Kabelbrände unterhalb der Verkleidung des Flugzeuginnenraumes lokalisieren.

Auch können mit einer mobilen Messvorrichtung 1 unbekannte Flüssigkeiten, z.B. in Form eine Pfütze, innerhalb des Flugzeuginnenraumes oder auch außerhalb des Flugzeuges, beispielsweise unterhalb der Flugzeugtriebwerke, untersucht werden.

Denkbar ist es auch, die Messvorrichtung 1 einzig oder zusätzlich mit einem Differential Mobility Spectrometer (DMS) oder Field Asymetric Ion Mobility Spectrometer (FAIMS-Spektrometer) oder anderen geeigneten Detektoren zur Detektion der untersuchten Gase einzusetzen.

### Liste der Bezugszeichen

- 1: Messvorrichtung
- 2: Förderpumpe
- 3: Ionenmobilitätsspektrometer
- 4: Rechner
- 5: Bedien- und Anzeigeeinheit
- 6: Gasleitung
- 7: Abluftstutzen
- 8: Gasleitung
- 9: Gasleitung
- 10: Ansaugstutzen
- 11: Leitung
- 12: Leitung
- 13: Photoionisationsdetektor
- 14: Metalloxidsensor
- 15: elektrochemische Zelle
- 16: Verteiler
- 17: Förderpumpe
- 18: Ansaugstutzen
- 19: Zuluftauslass
- 20: Flugzeuginnenraum
- 21: Flugzeugtriebwerk I
- 22: Flugzeugtriebwerk II
- 23: Flugzeugtriebwerk III
- 24: Flugzeugtriebwerk IV
- 25: Erkennung der Gasklasse
- 26: Ermittlung der Gas- oder Geruchsintensität für die Gasklasse A
- 27: Ermittlung der Gas- oder Geruchsintensität für die Gasklasse B
- 28: Ermittlung der Gas- oder Geruchsintensität für die Gasklasse C
- 29: Ermittlung der Gas- oder Geruchsintensität für die Gasklasse D

## Patentansprüche

1. Verfahren zur Detektion und Identifizierung von riechbaren Gasen in Flugzeuginnenräumen, wobei die Zuluft des Flugzeuginnenraumes (20) einem Ansaugkanal einer ein Ionenmobilitätsspektrometer umfassenden Messvorrichtung (1) zugeführt wird, **dadurch gekennzeichnet, dass** die Messergebnisse der Messvorrichtung (1) mittels mathematischer Methoden analysiert werden, und die mathematischen Methoden sich in eine Kalibrierung und eine Gasvermessung unterteilen, wobei
- die Kalibrierung eine Aufteilung des Messbereiches der Messvorrichtung (1) in Gasklassen und eine Zuordnung von Grenzwerten für jede Gasklasse umfasst, wobei die Kalibrierung für die Messvorrichtung (1) unbekannte Gerüche verwendet, wobei die Messvorrichtung (1) auf die unbekannten Gerüche trainiert wird und für die Zuordnung von Grenzwerten für jede Gasklasse eine Datenbank mit einer paarweisen Anordnung von verschiedenen Messsignalen der Messvorrichtung (1) und Geruchsschwellwerten erstellt wird,
- die Gasvermessung eine Erkennung der Gasklassen und eine Ermittlung der Gasintensität umfasst, wobei bei der Gasvermessung mit der Erkennung der Gasklassen eine Gasklasse ausgewählt wird, für die anschließend die Ermittlung der Gasintensität durchgeführt wird, wobei bei der Gasvermessung die ermittelten Messergebnisse der Messvorrichtung (1) mit den vorher aufgenommenen Messergebnissen verglichen und aus dem Vergleich Rückschlüsse auf das vermessene Gas gezogen werden, und
- durch eine Änderung des Ansaugkanals der Zuluft des Flugzeuginnenraumes (20), oder durch eine Änderung der Betriebsparameter eines Flugzeugtriebwerkes, Rückschlüsse auf den Wartungszustand eines im Ansaugbereich des Ansaugkanals befindlichen Flugzeugtriebwerkes geschlossen werden.

2. Verfahren nach Anspruch 1,
**dadurch gekennzeichnet, dass**
- die Aufteilung des Messbereiches der Messvorrichtung (1) in Gasklassen durch ein künstlich-neuronales Netz, insbesondere durch ein Kohonen-Netz, erfolgt und
- die Zuordnung von Grenzwerten für jede Gasklasse durch ein künstlich-neuronales Netz, insbesondere durch ein Multilayer-Perceptron-Netz, erfolgt.

3. Verfahren nach einem der vorhergehenden Ansprüche,
**dadurch gekennzeichnet, dass** das zu messende Gas durch eine Förderpumpe (2) angesaugt und durch Verteiler (16) aufgeteilt wird, dann parallel einem Ionenmobilitätsspektrometer (3), einer elektrochemischen Zelle (15), einem Photoionisationsdetektor (13) und/oder zwei Metalloxidsensoren (14) zugeführt wird.

4. Verfahren nach Anspruch 3,
**dadurch gekennzeichnet, dass** das zu messende Gas vor dem Erreichen des Ionenmobilitätsspektrometers (3), der elektrochemischen Zelle (15), des Photoionisationsdetektors (13) und der Metalloxidsensoren (14), alternativ auch nur vor dem Erreichen des Ionenmobilitätsspektrometers (3), mit einem Referenzgas vermischt wird, indem das Referenzgas in definierten Mengen dem zu messenden Gas zugeführt wird, wobei beispielsweise mit der höchst möglichen Flussmenge des Referenzgases angefangen wird und diese später reduziert wird.

5. Verfahren nach Anspruch 4,
**dadurch gekennzeichnet, dass** das Messsignal des Ionenmobilitätsspektrometers (3), der elektrochemischen Zelle (15), des Photoionisationsdetektors (13) und/oder der Metalloxidsensoren (14) zur Regelung der Dosierung des Referenzgases verwendet wird, indem:
- die relative Signalhöhe der Metalloxidsensoren (14) direkt genutzt wird, um den Gasfluss der Förderpumpe (17) kurzfristig zu erhöhen, falls die Messsignale der Metalloxidsensoren (14) zunehmen sollten, oder um den Gasfluss der Förderpumpe (17) kurzfristig zu verringern, falls das Messsignal des Detektors abnehmen sollte, und/oder
- aus dem Messsignal des Ionenmobilitätsspektrometers (3) die absoluten Bereiche der Gasflussmenge des Referenzgases einzustellen, indem die absoluten Signalhöhen während eines längeren Zeitraumes verwendet werden, um die grobe Einstellung, insbesondere eine Einstellung des maximalen und minimalen Bereichs der Fördermenge des Referenzgases des Gasflusses zu ermöglichen.

6. Verfahren nach Anspruch 5,
**dadurch gekennzeichnet, dass** das zu messende Gas über eine Kombination aus dem Ionenmobilitätsspektrometer (3) und dem Photoionisationsdetektor (13) zum Nachweis von Aromaten, und zusätzlichen mit der elektrochemischen Zelle (15) zum Nachweis einzelner Substanzen, wie z. B. Phosgen, und zusätzlichen zu den Metalloxidsensoren (14) zum Nachweis von z. B. Kohlenwasserstoffen oder Kohlenmonoxid geleitet werden.

## Claims

1. A method for detecting and identifying gases that can be smelled in airplane interior spaces, wherein the air supplied into the airplane interior space (20) is made to flow to a suction channel of a measuring device (1) comprising an ion mobility spectrometer,
**characterized in that** the measuring results of the measuring device (1) are analyzed by means of mathematical methods, said mathematical methods being divided into a calibration and a gas measurement, wherein
- said calibration comprises a division of the measuring range of the measuring device (1) into gas classes and an allocation of limits to each gas class, wherein the calibration uses smells that are not known to the measuring device (1), wherein the measuring device (1) is trained to said unknown smells, and a database where different measuring signals of the measuring device (1) and smell thresholds are arranged in pairs is compiled for the purpose of allocating limits to each gas class,
- said gas measurement comprises an identification of the gas classes and a determination of the gas intensity, wherein during the gas measurement a gas class is selected when the gas classes are identified, and then the gas intensity of the selected gas class is determined, wherein during the gas measurement the measuring results determined by the measuring device (1) are compared with the measuring results recorded earlier and said comparison serves to draw conclusions relating to the gas that has been measured, and
- conclusions relating to the state of maintenance of an airplane engine located in the suction area of the suction channel are made by changing the suction channel of the air supplied into the airplane interior space (20) or by changing the operating parameters of an airplane engine.

2. The method according to claim 1,
**characterized in that**
- the measuring range of the measuring device (1) is divided into gas classes by means of an artificial neural network, in particular a Kohonen network, and
- limits are allocated to each gas class by means of an artificial neural network, in particular a multilayer perceptron network.

3. The method according to any one of the preceding claims,
**characterized in that** the gas to be measured is drawn in by a feed pump (2) and distributed by distributors (16), and is then supplied in parallel to an ion mobility spectrometer (3), an electrochemical cell (15), a photoionization detector (13) and/or two metal oxide sensors (14).

4. The method according to claim 3,
**characterized in that** the gas to be measured is mixed with a reference gas before it reaches the ion mobility spectrometer (3), the electrochemical cell (15), the photoionization detector (13) and the metal oxide sensors (14), alternatively only before it reaches the ion mobility spectrometer (3), by supplying defined amounts of said reference gas to the gas to be measured, starting with the highest possible volumetric flow rate of the reference gas and reducing it later, for example.

5. The method according to claim 4,
**characterized in that** the measuring signal of the ion mobility spectrometer (3), the electrochemical cell (15), the photoionization detector (13) and/or the metal oxide sensors (14) is used to control the dosage of the reference gas by:
- directly using the relative signal level of the metal oxide sensors (14) in order to bring about a short-term increase in the gas flow of the feed pump (17) in case the measuring signals of the metal oxide sensors (14) should increase, or in order to bring about a short-term reduction in the gas flow of the feed pump (17) in case the measuring signal of the detector should decrease, and/or
- the measuring signal of the ion mobility spectrometer (3) is used to adjust the absolute ranges of the gas flow of the reference gas by using the absolute signal levels during a longer period of time in order that a rough adjustment, in particular an adjustment of the maximum and minimum ranges of the feed volume of the reference gas of the gas flow, can be made.

6. The method according to claim 5,
**characterized in that** the gas to be measured is made to flow across a combination of the ion mobility spectrometer (3) and the photoionization detector (13) in order to detect aromatic compounds, and in addition is made to flow to the electrochemical cell (15) in order to detect individual substances, such as phosgene, and in addition to the metal oxide sensors (14) in order to detect e.g. hydrocarbons or carbon monoxide.

## Revendications

1. Procédé de détection et d'identification de gaz perceptibles par l'odorat dans les espaces intérieurs d'aéronefs, l'air alimentant l'espace intérieur d'aéronefs (20) étant conduit à une gaine d'aspiration d'un dispositif de mesure (1) comprenant un spectromètre de mobilité ionique, **caractérisé en ce que** les résultats de mesure du dispositif de mesure (1) sont analysés au moyen de méthodes mathématiques, et **en ce que** les méthodes mathématiques se répartissent en un étalonnage et en une gazométrie,
- l'étalonnage comprenant une répartition de la plage de mesure du dispositif de mesure (1) en catégories de gaz et une affectation de valeurs limites pour chaque catégorie de gaz, l'étalonnage utilisant des odeurs inconnues pour le dispositif de mesure (1), le dispositif de mesure (1) étant formé aux odeurs inconnues, et une base de données étant, pour l'affectation de valeurs limites pour chaque catégorie de gaz, établie avec un agencement par paires de différents signaux de mesure du dispositif de mesure (1) et de valeurs de seuil d'odeurs,
- la gazométrie comprenant une identification des catégories de gaz et une détermination de l'intensité gazeuse, une catégorie de gaz étant sélectionnée lors de la gazométrie avec la détection des catégories de gaz et faisant ensuite l'objet de la détermination de l'intensité gazeuse, les résultats de mesure du dispositif de mesure (1) déterminés étant, lors de la gazométrie, comparés aux résultats de mesure enregistrés précédemment, et des conclusions concernant le gaz mesuré étant tirées à partir de la comparaison, et
- des conclusions concernant l'état de maintenance d'un moteur d'aéronef situé dans la zone d'aspiration de la gaine d'aspiration étant tirées par une modification de la gaine d'aspiration de l'air alimentant l'espace intérieur d'aéronefs (20) ou par une modification des paramètres de fonctionnement d'un moteur d'aéronef.

2. Procédé selon la revendication 1,
**caractérisé en ce que**
- la répartition de la plage de mesure du dispositif de mesure (1) en catégories de gaz est effectuée par un réseau de neurones artificiels, en particulier par un réseau de Kohonen, et
- l'affectation de valeurs limites pour chaque catégorie de gaz est effectuée par un réseau de neurones artificiels, en particulier par un réseau perceptron multicouche.

3. Procédé selon une des revendications précédentes,
**caractérisé en ce que** le gaz à mesurer est aspiré à travers une pompe d'alimentation (2) et réparti par des distributeurs (16), puis conduit en parallèle à un spectromètre de mobilité ionique (3), à une cellule électrochimique (15), à un détecteur à photoionisation (13) et/ou à deux capteurs à oxydes métalliques (14).

4. Procédé selon la revendication 3,
**caractérisé en ce que,**
avant d'atteindre le spectromètre de mobilité ionique (3), la cellule électrochimique (15), le détecteur à photoionisation (13) et les capteurs à oxydes métalliques (14), ou bien également uniquement avant d'atteindre le spectromètre de mobilité ionique (3), le gaz à mesurer est mélangé à un gaz de référence en conduisant le gaz de référence, en quantités définies, au gaz à mesurer, en commençant par exemple avec le débit du gaz de référence le plus élevé possible et en réduisant ultérieurement ce débit.

5. Procédé selon la revendication 4,
**caractérisé en ce que** le signal de mesure du spectromètre de mobilité ionique (3), de la cellule électrochimique (15), du détecteur à photoionisation (13) et/ou des capteurs à oxydes métalliques (14) est utilisé pour la régulation du dosage du gaz de référence en :
- utilisant directement la hauteur de signal relative des capteurs à oxydes métalliques (14) pour augmenter brièvement le flux de gaz de la pompe d'alimentation (17) si les signaux de mesure des capteurs à oxydes métalliques (14) viennent à augmenter, ou pour réduire brièvement le flux de gaz de la pompe d'alimentation (17) si le signal de mesure du détecteur vient à diminuer, et/ou
- pour régler, à partir du signal de mesure du spectromètre de mobilité ionique (3), les plages absolues du débit de gaz du gaz de référence en utilisant les hauteurs de signaux absolues pendant une période assez longue afin de permettre le réglage grossier, en particulier un réglage de la plage maximale et minimale du débit du gaz de référence du flux de gaz.

6. Procédé selon la revendication 5,
**caractérisé en ce que** le gaz à mesurer est conduit par le biais d'une combinaison constituée du spectromètre de mobilité ionique (3) et du détecteur à photoionisation (13) pour la mise en évidence d'aromates, et en plus avec la cellule électrochimique (15) pour la mise en évidence de substances individuelles, comme par exemple un phosgène, et en plus aux capteurs à oxydes métalliques (14) pour la mise en évidence par exemple d'hydrocarbures ou de monoxyde de carboné.
